# FASCICULE DE BREVET EUROPEEN

(11) **EP 0 976 413 B1**
(45) Date de publication et mention de la délivrance du brevet: **23.04.2008**
(21) Numéro de dépôt: 99401886.9
(22) Date de dépôt: 23.07.1999
(51) Int. Cl.: A61M 1/02

(54) **Ensemble de poches en circuit clos, destiné à recueillir, séparer et purifier les différents constituants du sang à partir d'un prélèvement de sang total**
Im geschlossenen Kreislauf angeordnetes Beutelsystem zum Sammeln, Trennen und Reinigen der verschiedenen Blutbestandteile aus Gesamtblut
Closed loop bag system for collection, separation and purification of blood constituents of a whole blood sample

(30) Priorité: 31.07.1998 FR 9809889
(43) Date de publication de la demande: 02.02.2000
(73) Titulaire: MACO PHARMA, F-59200 Tourcoing (Nord) (FR)
(72) Inventeur: Goudaliez, Francis, 59155 Faches Thumesnil (FR); Verpoort, Thierry, 59420 Mouvaux (FR); Vezon, Gérard, 33000 Bordeaux (FR)
(74) Mandataire: Derambure, Christian

(56) Documents cités:
- EP-A- 0 349 188
- EP-A- 0 591 980
- DE-A- 4 022 700
- FR-A- 2 677 883
- US-A- 5 100 564

## Description

L'invention se rapporte à un ensemble de poches pour le recueil des différents constituants du sang.

Il existe déjà des ensembles de poches, permettant de recueillir, de filtrer le sang total et de le séparer ensuite en ses différents constituants.

Ces ensembles de poches comprennent généralement une poche primaire de prélèvement reliée par l'intermédiaire d'un filtre, notamment un filtre à déleucocyter, à des poches secondaires ou satellites.

Deux types différents d'ensembles de poches sont utilisés, selon les constituants du sang que l'on souhaite obtenir.

Le premier type d'ensemble de poches permet le prélèvement, la filtration du sang total, puis après décantation, l'obtention d'un concentré d'érythrocytes déleucocytés d'une part et d'un plasma "acellulaire" d'autre part. Le document EP 349 188 décrit un tel ensemble.

Le second type d'ensemble de poches permet le prélèvement, puis après centrifugation et décantation, l'obtention d'un plasma riche en plaquettes permettant la préparation d'un concentré de plaquettes et d'un plasma pauvre en plaquettes, d'une part et, après filtration, l'obtention d'un concentré érythrocytaire déleucocyté d'autre part. Le document EP 591 980 décrit un tel ensemble.

Il n'existe cependant pas à l'heure actuelle de système unique avec filtre intégré permettant, après filtration du sang total, la préparation d'un concentré de plaquettes et d'un concentré érythrocytaire déleucocyté, suivant le mode opératoire utilisé.

L'invention a pour but de remédier à cet inconvénient.

Elle permet, après le prélèvement et en fonction des besoins de l'utilisateur, de choisir le type de préparations.

A cet effet, l'ensemble de poches selon l'invention, fonctionnant en circuit clos, comprend tout d'abord une poche primaire de recueil du sang.

La poche primaire est reliée, au niveau d'un orifice d'entrée, à un dispositif de prélèvement du sang, classique en lui-même.

La poche primaire est également reliée, au niveau d'un premier orifice de sortie, à la première extrémité d'une première tubulure communiquant, par son autre extrémité, avec l'orifice d'entrée d'un filtre, notamment un filtre à déleucocyter, permettant la filtration du sang total ou du concentré érythrocytaire.

Le filtre à déleucocyter communique par son orifice de sortie avec la première extrémité d'une seconde tubulure communiquant, par sa seconde extrémité, avec l'orifice d'entrée d'une première poche secondaire de recueil.

Selon l'invention, la poche primaire est reliée de plus, au niveau d'un second orifice de sortie, à la première extrémité d'une troisième tubulure.

Cette troisième tubulure communique par l'intermédiaire d'un premier système de dérivation, avec une deuxième poche secondaire de recueil, et par l'intermédiaire d'un deuxième système de dérivation, avec une troisième poche secondaire de recueil.

La troisième tubulure communique de plus, par sa seconde extrémité, avec la première poche secondaire de recueil.

Selon d'autres caractéristiques, les orifices de sortie de la poche primaire sont disposés au niveau de la partie supérieure de la poche primaire.

Selon une autre forme d'exécution de l'invention, les orifices de sortie de la poche primaire sont disposés, l'un dans la partie supérieure de la poche, l'autre dans la partie inférieure de la poche primaire.

L'ensemble de poches de l'invention permet de préparer, en utilisant le même ensemble de poches, et en modifiant simplement le mode opératoire utilisé, un concentré d'érythrocytes déleucocytés et un plasma riche en plaquettes à partir duquel un concentré de plaquettes et un plasma peuvent être obtenus, ou un concentré d'érythrocytes déleucocytés et un plasma "acellulaire".

Un seul et unique filtre permet à la fois la filtration du sang total ou d'un concentré érythrocytaire.

L'invention sera mieux comprise dans la description qui suit, faite en référence aux figures annexées.

La figure 1 représente une vue schématique d'un premier mode de réalisation de l'ensemble de poches de l'invention.

La figure 2 représente une vue schématique d'un autre mode de réalisation de l'ensemble de poches de l'invention.

En se référant maintenant aux figures, l'ensemble de poches 1 selon l'invention comprend une poche primaire 2 reliée au niveau d'un orifice d'entrée 3 à un dispositif de prélèvement du sang.

Le dispositif de prélèvement du sang est classique en lui-même et comprend généralement au moins une tubulure 4 et une aiguille de prélèvement 4a.

La poche primaire 2 est munie de deux orifices de sortie 5 et 6.

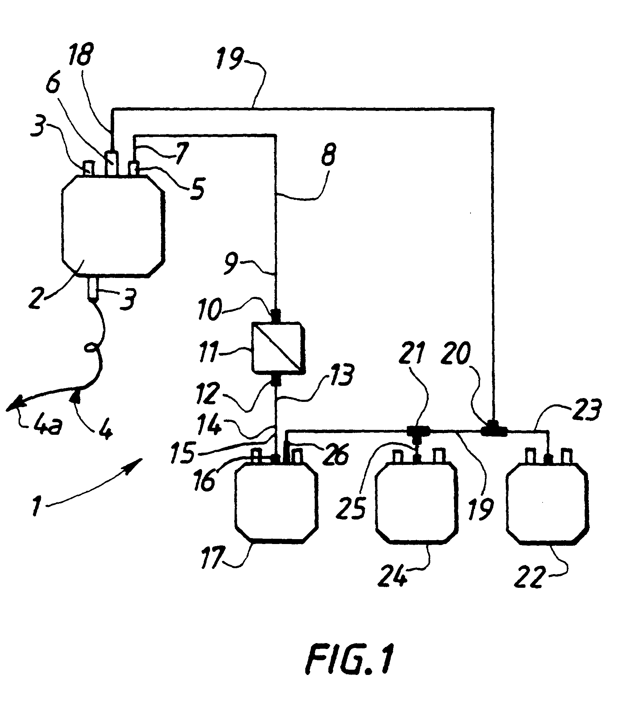

Sur le mode d'exécution représenté sur la figure 1, les deux orifices de sortie 5, 6 sont disposés au niveau de la partie supérieure de la poche primaire 2.

Par partie supérieure de la poche primaire 2, on entend dans ce qui suit la région de la poche primaire qui se trouve sur le dessus lorsque la poche est introduite verticalement dans le godet de centrifugation.

Le premier orifice de sortie 5 est relié à la première extrémité 7 d'une première tubulure 8 communiquant par son autre extrémité 9 avec l'orifice d'entrée 10 d'un filtre 11.

Le filtre 11 est généralement un filtre à déleucocyter, comprenant un milieu filtrant et permettant de retenir dans ce milieu filtrant la majeure partie des leucocytes et des plaquettes sanguines.

Le filtre à déleucocyter 11 peut par exemple consister en un filtre à déleucocyter tel que décrit dans le document EP-A-0 526 678, dont le contenu est incorporé ici par référence.

Un tel filtre à déleucocyter comprend notamment une poche filtrante munie d'une enveloppe extérieure souple formée par assemblage mutuel de la périphérie de deux feuilles de matière plastique.

Cette enveloppe extérieure renferme un milieu filtrant qui est maintenu dans un cadre souple étanche, délimitant deux compartiments, respectivement d'entrée et de sortie, de la poche filtrante.

Le sang arrive ainsi dans le compartiment d'entrée par un orifice d'entrée à travers le milieu filtrant, dans le compartiment de sortie et sort finalement de ce compartiment de filtration par un orifice de sortie.

Le matériau poreux utilisé pour le milieu filtrant peut par exemple comprendre un matériau hydrophile tel que la cellulose et ses dérivés, par exemple l'acétate de cellulose.

Il peut également comprendre un matériau tel qu'un polymère ou copolymère à base de polypropylène, polyester, polyamide, polyéthylène haute ou basse densité, polyuréthanne et leurs dérivés, rendu hydrophile par un traitement physique ou chimique conventionnel.

De tels traitements consistent par exemple dans le greffage de groupements hydrophiles, par exemple de type hydroxyle ou carboxylique, sur le polymère ou le copolymère.

Le caractère hydrophile permet le mouillage du milieu filtrant lors du passage du sang.

Le milieu filtrant peut comprendre une ou plusieurs couches, éventuellement de porosités et/ou de compositions différentes entre elles, de matériaux poreux, sous forme par exemple d'un non-tissé.

Généralement, lorsque le milieu filtrant comporte plusieurs couches de porosités différentes, les couches de plus grande porosité sont disposées de façon à ce que le sang passe en premier par les couches de plus grande porosité, puis par les couches de plus faible porosité.

Le milieu filtrant peut également comprendre un pré-filtre disposé du côté du compartiment d'entrée de filtration.

Ce pré-filtre sert à retenir les particules les plus grosses non désirables éventuellement présentes dans le sang.

Le pré-filtre peut comprendre une ou plusieurs couches, éventuellement de porosités et/ou de compositions différentes entre elles, d'un matériau poreux, sous forme par exemple d'un non-tissé.

Les matériaux hydrophiles ou rendus hydrophiles décrits ci-dessus pour la réalisation du milieu filtrant peuvent également être utilisés pour le pré-filtre.

Le filtre à déleucocyter 11 communique par son orifice de sortie 12 avec la première extrémité 13 d'une seconde tubulure 14, communiquant elle-même par son autre extrémité 15 avec l'orifice d'entrée 16 d'une première poche secondaire de recueil 17.

Le second orifice de sortie 6 de la poche primaire 2 communique avec la première extrémité 18 d'une troisième tubulure 19.

Cette troisième tubulure 19 comprend deux systèmes de dérivation 20, 21, se présentant par exemple sous la forme de raccord en Té.

Il va de soi que d'autres systèmes de dérivation peuvent être utilisés, par exemple des raccords en forme de Y.

Le premier système de dérivation 20 fait communiquer la troisième tubulure 19 avec une deuxième poche secondaire de recueil 22.

Une tubulure 23 peut éventuellement être prévue entre le système de dérivation 20 et la deuxième poche secondaire 22.

Le deuxième système de dérivation 21 met en communication la troisième tubulure 19 avec une troisième poche secondaire de recueil 24.

De la même façon, une tubulure 25 peut être prévue entre le système de dérivation 21 et la troisième poche secondaire 24.

La seconde extrémité de la troisième tubulure 19, opposée à la première extrémité 18, est quant à elle en communication, par l'intermédiaire d'un second orifice d'entrée 26, avec la première poche secondaire de recueil 17.

Il apparaît donc sur la figure 1 que les trois poches secondaires de recueil 17, 24, 22 sont en communication avec la troisième tubulure 19.

Selon le mode de réalisation représenté sur la figure 1, les orifices de sortie 5, 6 de la poche primaire 2 sont disposés au niveau de la partie supérieure de la poche 2.

Au moins une poche secondaire de recueil, par exemple la deuxième poche secondaire de recueil 22, contient une solution additive, telles que celles classiquement utilisées pour la conservation des érythrocytes.

Les tubulures peuvent être réalisées en un matériau souple et soudable, par exemple une matière plastique.

Les poches primaires et secondaires peuvent être des poches souples, réalisées également en une matière plastique conventionnellement utilisée pour ce type de produit.

Les différentes poches, filtres, et tubulures de l'ensemble de poches 1 de l'invention sont assemblées entre elles de façon fixe, de façon à fonctionner en circuit totalement clos.

On décrit ci-après sommairement le mode d'utilisation de l'ensemble de poches représenté sur la figure 1.

On considère en premier lieu le cas où l'on souhaite préparer, à partir du sang total, un concentré d'érythrocytes déleucocytés et un plasma "acellulaire".

Dans ce cas, le sang total arrive par la tubulure 4 dans la poche primaire 2.

Après obturation de la troisième tubulure 19, par exemple au moyen de systèmes de fermeture du type clamps, le sang passe, via la première tubulure 8, dans le filtre à déleucocyter 11, dans lequel la majeure partie des leucocytes et des plaquettes sanguines sont retenus.

Il est également envisageable que la poche primaire 2 comporte au niveau de ses orifices de sortie des éléments seccables permettant de diriger de façon préférentielle le sang.

Le sang déleucocyté passe ensuite, via la seconde tubulure 14, dans la première poche secondaire de recueil 17.

Les seconde tubulure 14 et troisième tubulure 19 sont ensuite obturées, par exemple par soudure, entre le filtre 11 et la première poche secondaire de recueil 17, et entre la poche primaire 2 et les poches secondaires 22 et 24.

La poche 2 et le filtre 1 qui ont rempli leur office sont éliminés.

La première poche secondaire de recueil 17 est ensuite soumise à une centrifugation, avec l'ensemble des autres poches, de façon à séparer le plasma et les érythrocytes.

Le plasma est envoyé, via la troisième tubulure 19, dans la troisième poche secondaire de recueil 24.

La solution additive de conservation des érythrocytes initialement contenue dans la deuxième poche secondaire de recueil 22 est envoyée dans la première poche secondaire de recueil 17.

La troisième tubulure 19 peut être alors obturée entre la poche secondaire de recueil 17 contenant des érythrocytes et la poche secondaire de recueil 24 contenant le plasma.

On envisage maintenant le cas où l'on souhaite préparer à partir du sang total un concentré érythrocytaire déleucocyté, un plasma et un concentré de plaquettes.

Dans ce cas, le sang est prélevé par l'intermédiaire de la tubulure 4 et arrive dans la poche primaire 2.

L'ensemble de poches 1 est alors soumis à une centrifugation, de façon à séparer dans la poche primaire 2 les constituants sanguins.

Le plasma riche en plaquettes est envoyé, après rupture de l'obturateur interne, de la sortie 6 de la tubulure 18 via la troisième tubulure 19, dans la troisième poche secondaire de recueil 24.

La solution additive contenue initialement dans la deuxième poche secondaire de recueil 22 est envoyée, via la troisième tubulure 19, dans la poche primaire 2.

Les tubulures sont ensuite obturées entre les poches primaire 2 et de recueil 22, et 17 et 24. L'ensemble 2, 11, 17 est séparé de l'ensemble 22, 24.

Le concentré d'érythrocytes, resuspendu dans la solution additive, contenu dans la poche primaire 2 est passé, via la première tubulure 8, dans le filtre à déleucocyter 11, et, par l'intermédiaire de la seconde tubulure 14, dans la première poche secondaire de recueil 17.

Une seconde centrifugation des poches secondaires de recueil 24 et 22 est réalisée.

Le plasma surnageant est envoyé dans la poche secondaire de recueil 22, le concentré de plaquettes restant dans la troisième poche secondaire de recueil 24.

Les tubulures entre les poches secondaires de recueil 22 et 24 sont obturées.

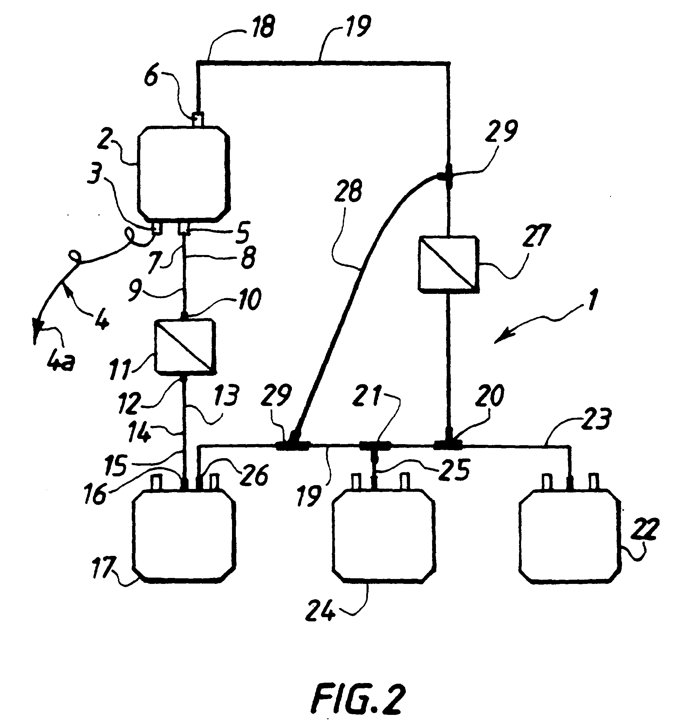

On décrit maintenant dans ce qui suit un second mode de réalisation de l'ensemble de poches de l'invention, en référence plus particulièrement avec la figure 2.

Selon ce mode de réalisation, l'ensemble de poches 1 de l'invention comprend une poche primaire 2 communiquant par son orifice d'entrée 3 avec une tubulure 4 reliée à une aiguille de prélèvement 4a.

La poche primaire 2 comporte deux orifices de sortie 5, 6, l'orifice de sortie 6 étant disposé au niveau de la partie supérieure de la poche primaire 2, le second orifice de sortie 5 étant disposé au niveau de la partie inférieure de la poche primaire 2.

L'orifice de sortie 5, situé à la partie inférieure de la poche primaire 2 communique avec la première extrémité 7 d'une première tubulure 8.

La seconde extrémité 9 de la tubulure 8 communique quant à elle avec l'orifice d'entrée 10 d'un filtre 11, notamment à déleucocyter.

Le filtre 11 est muni d'un orifice de sortie 12 communiquant avec la première extrémité 13 d'une seconde tubulure 14.

L'autre extrémité 15 de la seconde tubulure 14 communique avec l'orifice d'entrée 16 d'une première poche secondaire de recueil 17.

Le second orifice de sortie 6 de la poche de prélèvement 2 communique avec une troisième tubulure 19, par l'intermédiaire de sa première extrémité 18.

La troisième tubulure 19 est munie d'un système de dérivation 20, par exemple en forme de Té, permettant de mettre en communication la troisième tubulure 19 avec une deuxième poche secondaire de recueil 22.

La troisième tubulure 19 est munie d'un second système de dérivation 21, par exemple en Té, permettant la communication avec une troisième poche secondaire de recueil 24.

De la même façon que pour le mode de réalisation précédent, des tubulures 23 et 25 peuvent être disposées entre le raccord 20 et la deuxième poche secondaire de recueil 22 et le raccord 21 et la troisième poche de recueil 22 respectivement.

La seconde extrémité de la troisième tubulure 19, opposée à la première extrémité 18, est quant à elle en communication par l'intermédiaire d'un second orifice d'entrée 26 avec la poche secondaire de recueil 17.

Un second filtre 27 est disposé entre la poche primaire de prélèvement 2 et la seconde poche secondaire de recueil 22.

Ce second filtre 27 est notamment un filtre à plasma, classique en lui-même.

Une tubulure 28 relie par l'intermédiaire de raccords 29, la région de la troisième tubulure 19 située du côté de la première poche secondaire de recueil 17 et la région de la tubulure 19 située en amont du second filtre 27.

Les matériaux constitutifs et l'assemblage en lui-même de ce second mode de réalisation peuvent être conformes à ceux décrits pour le premier mode de réalisation ci-dessus.

On décrit maintenant sommairement les conditions d'utilisation de ce second mode d'exécution de l'invention.

On considère en premier lieu le cas où l'on souhaite préparer un concentré d'érythrocytes déleucocytés d'une part et un plasma d'autres part.

Dans ce cas, le sang total arrive par la tubulure 4 dans la poche primaire de prélèvement 2.

Le sang est filtré par passage dans le filtre à déleucocyter 11, par l'intermédiaire de la première tubulure 8.

Le sang débarrassé de la plus grande partie des leucocytes et des plaquettes sanguines est ensuite envoyé, par l'intermédiaire de la seconde tubulure 14, dans la première poche secondaire de recueil 17.

La première poche secondaire de recueil 17 est ensuite soumise à une centrifugation.

Après obturation de la tubulure 19 entre le raccord 29 et le raccord 21, le plasma est envoyé, par l'intermédiaire de la tubulure 28 et en passant par le second filtre 27, dans la troisième poche secondaire de recueil 24.

La solution additive contenue dans la deuxième poche secondaire de recueil 22 est envoyée, par l'intermédiaire de la tubulure 19, dans la première poche secondaire de recueil 17.

On considère maintenant le cas où l'on souhaite préparer un concentré érythrocytaire déleucocyté d'une part et un concentré plaquétaire et un plasma déleucocyté d'autre part.

Dans ce cas, le sang arrive par la tubulure 4 dans la poche primaire 2..

L'ensemble des poches est soumis à une centrifugation.

Suivant les conditions dans lesquelles est réalisée la centrifugation, on distingue dans la poche primaire 2 une première partie, localisée au niveau de la partie inférieure de la poche 2, comprenant essentiellement les érythrocytes et une seconde région, localisée à la partie supérieure de la poche primaire 2, comprenant essentiellement du plasma.

Entre ces deux régions, une zone tampon, encore appelée "buffycoat" ou couche leucoplaquétaire apparaît, comprenant essentiellement des plaquettes sanguines et une grande partie des leucocytes.

La partie supérieure contenue dans la poche 2, correspondant au plasma, est envoyée, par l'intermédiaire de la troisième tubulure 19, dans la troisième poche secondaire de recueil 24.

Lors de ce passage, le plasma passe par le second filtre 27 dans lequel la totalité des cellules est retenue.

Le plasma recueilli dans la troisième poche secondaire de recueil 24 est donc acellulaire.

La solution additive contenue initialement dans la seconde poche secondaire de recueil 22 est envoyée par exemple par l'intermédiaire de la tubulure 19, dans la première poche secondaire de recueil 17.

Le concentré érythrocytaire présent dans la partie inférieure de la poche primaire 2 est alors envoyé, par l'intermédiaire des tubulures 8 et 14 et en passant par le filtre 11, dans la première poche secondaire de recueil 17.

La première poche secondaire de recueil 17 contient alors un concentré érythrocytaire déleucocyté.

La couche leucoplaquétaire restant dans la poche primaire 2 peut alors servir à la préparation d'un concentré de plaquettes.

## Revendications

1. Ensemble de poches (1), fonctionnant en circuit clos, comprenant une poche primaire (2) de recueil du sang reliée au niveau d'un orifice d'entrée (3) à un dispositif de prélèvement du sang (4, 5), la poche primaire (2) étant reliée, au niveau d'un premier orifice de sortie (5), à la première extrémité (7) d'une première tubulure (8) communiquant par son autre extrémité (9) avec l'orifice d'entrée (10) d'un filtre à déleucocyter (11), le filtre à déleucocyter (11) communiquant par son orifice de sortie (12) avec la première extrémité (13) d'une seconde tubulure (14) communiquant par son autre extrémité (15), avec l'orifice d'entrée (16) d'une première poche secondaire de recueil (17), une troisième tubulure (19), communiquant par sa seconde extrémité avec la première poche secondaire de recueil (17), et communiquant, par l'intermédiaire d'un premier système de dérivation, avec une deuxième poche secondaire de recueil (22), et par l'intermédiaire d'un deuxième système de dérivation, avec une troisième poche secondaire de recueil (24),**caractérisé en ce que** la troisième tubulure (19) est branchée par sa première extrémité (18) sur la poche primaire (2) au niveau d'un second orifice de sortie (6) et **en ce que** le filtre à déleucocyter (11) permet à la fois la filtration du sang total ou d'un concentré érythrocytaire.

2. Ensemble de poches selon la revendication 1, **caractérisé en ce que** les orifices de sortie (5, 6) de la poche primaire (2) sont disposés au niveau de la partie supérieure de la poche primaire (2).

3. Ensemble de poches selon la revendication 1, **caractérisé en ce que** les orifices de sortie (5, 6) de la poche primaire (2) sont disposés, l'un dans la partie supérieure de la poche primaire (2), l'autre dans la partie inférieure de la poche primaire (2).

4. Ensemble de poches selon la revendication 3, **caractérisé en ce que** l'orifice de sortie (6) de la poche primaire (2) disposé dans la partie supérieure de la poche (2) est relié à l'extrémité (18) de la troisième tubulure (19).

5. Ensemble de poches selon la revendication 3 ou 4, **caractérisé en ce que** l'orifice de sortie (5) de la poche primaire (2) disposé dans la partie inférieure de la poche (2) est relié à la première extrémité (7) de la première tubulure (8).

6. Ensemble de poches selon l'une quelconque des revendications 3 à 5, **caractérisé en ce qu'**il comprend un second filtre (27) communiquant par son orifice d'entrée avec l'extrémité de la troisième tubulure (19) en communication avec la poche primaire (2) et par son orifice de sortie avec l'extrémité de la troisième tubulure (19) en communication avec les deuxième et troisième poches secondaires de recueil (22, 24).

7. Ensemble de poches selon la revendication 6, **caractérisé en ce que** le second filtre (27) est un filtre à plasma.

8. Ensemble de poches selon l'une quelconque des revendications 3 à 7, **caractérisé en ce qu'**il comprend une tubulure (28) reliant, par l'intermédiaire de raccords (29), la région de la troisième tubulure (19) située du côté de la première poche secondaire de recueil (17) et la région de la tubulure (19) située en amont du second filtre (27).

9. Ensemble de poches selon l'une quelconque des revendications 1 à 8, **caractérisé en ce que** le filtre à déleucocyter (11) comprend un milieu filtrant consistant en une ou plusieurs couches d'un matériau poreux, par exemple un matériau hydrophile tel que la cellulose et ses dérivés, par exemple l'acétate de cellulose, un matériau tel qu'un polymère ou copolymère à base de polypropylène, polyester, polyamide, polyéthylène haute ou basse densité, polyuréthanne et leurs dérivés, rendu hydrophile par un traitement physique ou chimique.

10. Ensemble de poches selon la revendication 9, **caractérisé en ce que** le milieu filtrant comprend une ou plusieurs couches, éventuellement de porosités et/ou de compositions différentes entre elles, de matériaux poreux, sous forme par exemple d'un non-tissé.

11. Ensemble de poches selon la revendication 9 ou 10, **caractérisé en ce que** le milieu filtrant comprend un pré-filtre disposé du côté du compartiment d'entrée de filtration.

12. Ensemble de poches selon la revendication 11, **caractérisé en ce que** le pré-filtre comprend une ou plusieurs couches, éventuellement de porosités et/ou de compositions différentes entre elles, d'un matériau poreux, sous forme par exemple d'un non-tissé.

13. Ensemble de poches selon l'une quelconque des revendications 1 à 12, **caractérisé en ce que** les tubulures sont obturables et soudables.

14. Ensemble de poches selon l'une quelconque des revendications 1 à 13, **caractérisé en ce qu'**au moins une poche secondaire de recueil (22) contient une solution de conservation des érythrocytes.

15. Ensemble de poches selon l'une quelconque des revendications 1 à 14, **caractérisé en ce que** les poches primaire (2) et secondaires (17, 22, 24) sont des poches souples, réalisées en une matière plastique souple.

16. Ensemble de poches selon l'une quelconque des revendications 1 à 15, **caractérisé en ce que** les systèmes de dérivation (20, 21) comprennent des raccords en Té ou en Y.

## Claims

1. A set of bags (1), functioning in closed circuit, comprising a primary bag (2) for receiving blood connected at an inlet orifice (3) to a blood take-off device (4, 5), the primary bag (2) being connected, at a first output orifice (5), to the first end (7) of a first tube (8) communicating through its other end (9) with the input orifice (10) of a leucocyte removal filter (11), the leucocyte removal filter (11) communicating through its outlet orifice (12) with the first end (13) of a second tube (14) communicating through its other end (15) with the inlet orifice (16) of a first secondary receiving bag (17), a third tube (19), communicating through its second end with the first secondary receiving bag (17), and communicating, by means of a first branch system, with a second secondary receiving bag (22), and by means of a second branch system, with a third secondary receiving bag (24), **characterised in that** the third tube (19) is connected through its first end (18) to the primary bag (2) at a second outlet orifice (6), and **in that** the leucocyte removal filter (11) allows filtration of both whole blood or of an erythrocytic concentrate.

2. A set of bags according to Claim 1, **characterised in that** the outlet orifices (5, 6) of the primary bag (2) are disposed at the top part of the primary bag (2).

3. A set of bags according to Claim 1, **characterised in that** the outlet orifices (5, 6) of the primary bag (2) are disposed, one in the top part of the primary bag (2), the other in the bottom part of the primary bag (2).

4. A set of bags according to Claim 3, **characterised in that** the outlet orifice (6) of the primary bag (2) disposed at the top part of the bag (2) is connected to the end (18) of the third tube (19).

5. A set of bags according to Claim 3 or 4, **characterised in that** the outlet orifice (5) of the primary bag (2) disposed in the bottom part of the bag (2) is connected to the first end (7) of the first tube (8).

6. A set of bags according to any one of Claims 3 to 5, **characterised in that** it comprises a second filter (27) communicating through its inlet orifice with the end of the third tube (19) communicating with the primary bag (2) and through its outlet orifice with the end of the third tube (19) communicating with the second and third secondary receiving bags (22, 24).

7. A set of bags according to Claim 6, **characterised in that** the second filter (27) is a plasma filter.

8. A set of bags according to any one of Claims 3 to 7, **characterised in that** it comprises a tube (28) connecting, by means of couplings (29), the region of the third tube (19) situated alongside the first secondary receiving bag (17) and the region of the tube (19) situated upstream from the second filter (27).

9. A set of bags according to any one of Claims 1 to 8, **characterised in that** the leucocyte removal filter (11) comprises a filtering medium consisting of one or more layers of a porous material, for example a hydrophilic material such as cellulose or derivatives thereof, for example cellulose acetate, a material such as a polymer or copolymer based on polypropylene, polyester, polyamide, high or low density polyethylene, polyurethane and their derivatives, made hydrophilic by a physical or chemical treatment.

10. A set of bags according to Claim 9, **characterised in that** the filtering medium comprises one or more layers, possibly with porosities and/or compositions different from each other, of porous materials, in the form of a non-woven material.

11. A set of bags according to Claim 9 or 10, **characterised in that** the filtering medium comprises a prefilter disposed alongside the filtration inlet compartment.

12. A set of bags according to Claim 11, **characterised in that** the prefilter comprises one or more layers, possibly with porosities and/or compositions different from each other, of a porous material, in the form for example of a non-woven material.

13. A set of bags according to any one of Claims 1 to 12, **characterised in that** the tubes can be closed off and welded.

14. A set of bags according to any one of Claims 1 to 13, **characterised in that** at least one secondary receiving bag (22) contains a solution for preserving erythrocytes.

15. A set of bags according to any one of Claims 1 to 14, **characterised in that** the primary (2) and secondary (17, 22, 24) bags are flexible bags, produced from a flexible plastics material.

16. A set of bags according to any one of Claims 1 to 15, **characterised in that** the branch systems (20, 21) comprise T or Y couplings.

## Patentansprüche

1. In geschlossenem Kreis funktionierende Beuteleinheit (1) mit einem Primär-Blutsammlungsbeutel (2), der über eine Eingangsöffnung (3) mit einer Blutabnahmevorrichtung (4, 5) verbunden ist, wobei der Primärbeutel (2) über eine erste Ausgangsöffnung (5) mit dem ersten Ende (7) eines ersten Stutzens (8) verbunden ist, dessen anderes Ende (9) mit der Eingangsöffnung (10) eines Deleukozyten-Filters (11) in Verbindung steht, wobei der Deleukozyten-Filter (11) über seine Ausgangsöffnung (12) mit dem ersten Ende (13) eines zweiten Stutzens (14) verbunden ist, dessen anderes Ende (15) mit der Eingangsöffnung (16) eines ersten Sekundärsammlungsbeutels (17) in Verbindung steht, wobei ein dritter Stutzen (19) über sein zweites Ende mit dem ersten Sekundärsammlungsbeutel (17) verbunden ist, und die über ein ersten Abzweigungssystem mit einem zweiten Sekundärsammelbeutel (22) und über ein zweites Abzweigungssystem mit einem dritten Sekundärsammelbeutel (24 verbunden ist, **dadurch gekennzeichnet, dass** der dritte Stutzen (19) mit seinem ersten Ende (18) am Primärbeutel (2) an einer zweiten Ausgangsöffnung (6) angeschlossen ist, und dass der Deleukozyten-Filter (11) gleichzeitig das Filtern des gesamten Blutes und eines erythrozytären Konzentrats ermöglicht.

2. Beuteleinheit nach Anspruch 1, **dadurch gekennzeichnet, dass** die Ausgangsöffnungen (5, 6) des Primärbeutels (2) am oberen Teil des Primärbeutels (2) angeordnet sind.

3. Beuteleinheit nach Anspruch 1, **dadurch gekennzeichnet, dass** die Ausgangsöffnungen (5, 6) des Primärbeutels (2) eine am oberen Teil des Primärbeutels (2) und die andere am unteren Teil des Primärbeutels (2) angeordnet sind.

4. Beuteleinheit nach Anspruch 3, **dadurch gekennzeichnet, dass** die am oberen Teil des Beutels (2) angeordnete Ausgangsöffnung (6) des Primärbeutels (2) mit dem Ende (18) des dritten Stutzens (19) verbunden ist.

5. Beuteleinheit nach Anspruch 3 oder 4, **dadurch gekennzeichnet, dass** die am unteren Teil des Beutels (2) angeordnete Ausgangsöffnung (5) des Primärbeutels (2) mit dem ersten Ende (7) des ersten Stutzens (8) verbunden ist.

6. Beuteleinheit nach einem der Ansprüche 3 bis 5, **dadurch gekennzeichnet, dass** sie einen zweiten Filter (27) umfasst, der über seine Eingangsöffnung mit dem Ende des dritten Stutzens (19) verbunden ist, der mit dem Primärbeutel (2) in Verbindung steht, und über seine Ausgangsöffnung mit dem Ende des dritten Stutzens (19), der mit dem zweiten und dritten Sekundärsammlungsbeutel (22, 24) verbunden ist.

7. Beuteleinheit nach Anspruch 6, **dadurch gekennzeichnet, dass** der zweite Filter (27) ein Plasmafilter ist.

8. Beuteleinheit nach einem der Ansprüche 3 bis 7, **dadurch gekennzeichnet, dass** die einen Stutzen (28) umfasst, der über Anschlüsse (29) die Region des dritten Stutzens (19) auf der Seite des ersten Sekundärsammlungsbeutels (17) und die Region des Stutzens (19) vor dem zweiten Filter (27) verbindet.

9. Beuteleinheit nach einem der Ansprüche 1 bis 8, **dadurch gekennzeichnet, dass** der Deleukozyten-Filter (11) ein Filtermilieu umfasst, das aus einer oder mehreren Schichten eines porösen Materials besteht, beispielsweise aus einem hydrophilen Material wie Zellulose und ihre Derivate, beispielsweise Zelluloseazetat, aus einem Material wie ein Polymer oder ein Copolymer auf Basis von Polypropylen, Polyester, Polyamid, Polyethylen mit hoher oder niedriger Dichte, Polyurethan und seinen Derivaten, das durch physikalische oder chemische Behandlung hydrophil gemacht wurde.

10. Beuteleinheit nach Anspruch 9, **dadurch gekennzeichnet, dass** das Filtermilieu eine oder mehrere Schichten von porösen Materialien umfasst, die eventuell verschiedene Porositäten und/oder Zusammensetzungen aufweisen, beispielsweise in Form eines Vliesstoffes.

11. Beuteleinheit nach Anspruch 9 oder 10, **dadurch gekennzeichnet, dass** das Filtermilieu einen Vorfilter umfasst, der neben dem Filterungseingangsabteil angeordnet ist.

12. Beuteleinheit nach Anspruch 11, **dadurch gekennzeichnet, dass** der Vorfilter eine oder mehrere Schichten von porösen Materialen umfasst, die eventuell verschiedene Porositäten und/oder Zusammensetzungen aufweisen, beispielsweise in Form eines Vliesstoffes.

13. Beuteleinheit nach einem der Ansprüche 1 bis 12, **dadurch gekennzeichnet, dass** die Stutzen verschliessbar und verschweissbar sind.

14. Beuteleinheit nach einem der Ansprüche 1 bis 13, **dadurch gekennzeichnet, dass** mindestens ein Sekundärsammlungsbeutel (22) eine Konservierungslösung der Erythrozyten aufweist.

15. Beuteleinheit nach einem der Ansprüche 1 bis 14, **dadurch gekennzeichnet, dass** der Primärbeutel (2) und die Sekundärbeutel (17, 22, 24) flexible Beutel aus einem flexiblen Kunststoff sind.

16. Beuteleinheit nach einem der Ansprüche 1 bis 15, **dadurch gekennzeichnet, dass** die Abzweigungssysteme (20, 21) T- oder Y-förmige Anschlüsse aufweisen.
